# FASCICULE DE BREVET EUROPEEN

(11) **EP 3 116 422 B1**
(45) Date de publication et mention de la délivrance du brevet: **03.01.2018**
(21) Numéro de dépôt: 15717538.1
(22) Date de dépôt: 10.03.2015
(51) Int. Cl.: A61B 17/72, A61B 17/68, A61B 17/88

(54) **IMPLANT CHIRURGICAL POUR UNE FUSION ENTRE DEUX PORTIONS D'OS ET ANCILLAIRE DE SERRAGE POUR SERRER UN TEL IMPLANT CHIRURGICAL**
CHIRURGISCHES IMPLANTAT ZUR ZUSAMMENFÜHRUNG VON ZWEI KNOCHENABSCHNITTEN UND HEFTHILFE ZUM HEFTEN SOLCH EINES CHIRURGISCHEN IMPLANTATS
SURGICAL IMPLANT FOR MERGING TWO BONE PORTIONS, AND CLAMPING AID FOR CLAMPING SUCH A SURGICAL IMPLANT

(30) Priorité: 11.03.2014 FR 1451980
(43) Date de publication de la demande: 18.01.2017
(73) Titulaire: Novastep, 35760 St Gregoire (FR)
(72) Inventeur: GIROD, Loïc, 35580 Goven (FR); GLEDEL, Grégory, 75011 Paris (FR); AUDIC, Gilles, 35137 Pleumeleuc (FR); AUGOYARD, Marc, 69160 Tassin la Demi Lune (FR); AUGOYARD, Romain, 69190 Tassin la Demi Lune (FR); MEUSNIER, Tristan, 42000 Saint Etienne (FR); VALENTIN, Stéphanie, 69002 Lyon (FR)
(74) Mandataire: Chevalier, Renaud Philippe
(86) Numéro de dépôt international: PCT/FR2015/050591
(87) Numéro de publication internationale: WO 2015/136212

(56) Documents cités:
- FR-A1- 2 856 269
- FR-A1- 2 901 119
- US-A1- 2011 301 653
- US-A1- 2012 083 791

## Description

La présente invention concerne un implant chirurgical destiné à permettre une fusion entre une première portion d'os et une deuxième portion d'os. De plus, la présente invention concerne un ancillaire de serrage, configuré pour serrer un implant chirurgical. De manière connue, un ancillaire est un instrument de manipulation destiné à aider le personnel médical.

La présente invention peut s'appliquer au domaine des implants chirurgicaux utilisés pour réaliser une arthrodèse par fusion osseuse intramédullaire dans une articulation. La présente invention peut aussi s'appliquer au domaine des implants chirurgicaux utilisés pour une osthéosynthèse par fusion entre deux fragments osseux. Les indications sont par exemple :
- la correction des orteils en griffe par arthrodèse de l'articulation interphalangienne proximale et/ou distale du pied,
- le traitement chirurgical de l'arthrose ou de traumatismes par arthrodèses des articulations interphalangiennes proximale et distale du pied,
- le traitement chirurgical de l'arthrose ou de traumatismes par arthrodèses des articulations interphalangiennes proximale et distale de la main,
- une osthéosynthèse par fusion de deux fragments osseux, en particulier du pied ou de la main, FR2901119 est considéré comme l'état de la technique le plus proche et illustre un implant chirurgical conforme au préambule de la revendication 1. FR2846545A1 illustre un implant chirurgical qui est en alliage à mémoire de forme. Cet implant chirurgical comprend deux parties d'ancrage, configurées pour ancrer respectivement dans un premier os et dans un deuxième os, et une partie intermédiaire reliant les parties d'ancrage entre elles. Les branches de chaque partie d'ancrage sont déplaçables entre :
   - une position d'introduction, dans laquelle les branches sont proches et peuvent être introduites dans un os respectif, l'implant chirurgical ayant alors globalement la forme d'un « H », et
   - une position d'ancrage, dans laquelle les branches sont plus écartées qu'en position d'introduction, de sorte que les branches ancrent chaque partie d'ancrage dans un os respectif, l'implant chirurgical ayant alors globalement la forme d'un « X ».

Cependant, comme l'implant chirurgical de FR2846545A1 est composé d'un alliage à mémoire de forme, il est nécessaire de le réfrigérer durant plusieurs heures afin de le placer en position d'introduction avant son implantation. Puis, le chirurgien n'a qu'un court laps de temps pour implanter cet implant chirurgical avant que ses branches ne reviennent, par l'effet de la mémoire de forme, en position d'ancrage. Donc, ce court laps de temps risque d'altérer la précision du positionnement de l'implant chirurgical dans les os. De plus, plus ce laps de temps est long, plus l'implant chirurgical recouvre peu à peu sa forme définitive, ce qui rend difficile voire impossible l'insertion de l'implant chirurgical au niveau du site osseux.

La présente invention a notamment pour but de résoudre, en tout ou partie, les problèmes mentionnés ci-avant.

Dans ce but, l'invention a pour objet un implant chirurgical, destiné à permettre une fusion entre une première portion d'os et une deuxième portion d'os, l'implant chirurgical comprenant au moins :
- une première partie d'ancrage configurée pour être ancrée dans la première portion d'os, la première partie d'ancrage comportant au moins deux premières branches, les premières branches étant déplaçables entre :
   - une position d'introduction, dans laquelle les extrémités respectives des premières branches définissent une première distance d'introduction, de façon à permettre l'introduction des premières branches à l'intérieur de la première portion d'os, et
   - une position d'ancrage, dans laquelle les extrémités respectives des premières branches définissent une première distance d'ancrage plus grande que la première distance d'introduction, de sorte que les premières branches sont adaptées pour ancrer la première partie d'ancrage à l'intérieur de la première portion d'os,
- une deuxième partie d'ancrage configurée pour être ancrée dans la deuxième portion d'os,
- une partie intermédiaire reliant la première partie d'ancrage à la deuxième partie d'ancrage ;
l'implant chirurgical étant caractérisé en ce que :
- la partie intermédiaire comporte au moins :
   - un évidement,
   - une première portion de liaison disposée entre la première partie d'ancrage et la deuxième partie d'ancrage, et
   - une deuxième portion de liaison disposée entre la première partie d'ancrage et la deuxième partie d'ancrage, la première portion de liaison et la deuxième portion de liaison étant agencées de part et d'autre dudit au moins un évidement de sorte que la première portion de liaison est distante de la deuxième portion de liaison, et
- la partie intermédiaire est conformée de sorte qu'un rapprochement de la première portion de liaison et de la deuxième portion de liaison déplace les premières branches de leur position d'introduction à leur position d'ancrage.

En d'autres termes, le rapprochement de la première portion de liaison et de la deuxième portion de liaison réduit ledit au moins un évidement, ce qui déplace les premières branches.

Avantageusement, ledit au moins un évidement présente un contour fermé. Alternativement, ledit au moins un évidement peut présenter un contour partiellement fermé ; par exemple, ledit au moins un évidement peut être ouvert sur 10 ou 20% de sa périphérie.

Selon une variante de l'invention, la première portion de liaison s'étend substantiellement entre la première branche et la deuxième partie d'ancrage, et la deuxième portion de liaison s'étend substantiellement entre la deuxième branche et la deuxième partie d'ancrage.

Selon une variante de l'invention, la première distance d'introduction peut être nulle ou négligeable.

Selon un mode de réalisation de l'invention, la deuxième partie d'ancrage comporte au moins deux deuxièmes branches, les deuxièmes branches étant déplaçables entre :
- une position d'introduction, dans laquelle les extrémités respectives des deuxièmes branches définissent une deuxième distance d'introduction, de façon à permettre l'introduction des deuxièmes branches à l'intérieur de la deuxième portion d'os, et
- une position d'ancrage, dans laquelle les extrémités respectives des deuxièmes branches définissent une deuxième distance d'ancrage plus grande que la deuxième distance d'introduction, de sorte que les deuxièmes branches sont adaptées pour ancrer la deuxième partie d'ancrage à l'intérieur de la deuxième portion d'os,

et dans lequel la partie intermédiaire est conformée de sorte qu'un rapprochement de la première portion de liaison et de la deuxième portion de liaison déplace les deuxièmes branches de leur position d'introduction à leur position d'ancrage.

En d'autres termes, l'implant chirurgical a au moins quatre branches, à raison d'au moins deux de chaque côté de la partie intermédiaire. Ainsi, un tel implant chirurgical permet un ancrage ferme et simple non seulement dans la première portion d'os, mais aussi dans la deuxième portion d'os.

Selon une variante de l'invention, la deuxième distance d'introduction peut être nulle ou négligeable.

Alternativement, la deuxième partie d'ancrage comporte une unique branche configurée pour être ancrée dans la deuxième portion d'os. Cette branche unique peut par exemple être formée par une tige d'ancrage.

Selon une variante de l'invention, les premières branches sont plus longues que les deuxièmes branches. Par exemple, le rapport entre une longueur des premières branches et une longueur des deuxièmes branches est compris entre 1,5 et 5. La longueur de chaque première branche peut être comprise entre 5 mm et 20 mm. La longueur de chaque deuxième branche peut être comprise entre 3 mm et 10 mm. La partie intermédiaire peut avoir une longueur, mesurée suivant la direction longitudinale d'une première branche, comprise entre 3 mm et 10 mm.

Selon une variante de l'invention, la partie intermédiaire, la première partie d'ancrage et la deuxième partie d'ancrage ont des épaisseurs comprises entre 0,5 mm et 2 mm.

Selon une variante de l'invention, la première partie d'ancrage comporte au moins trois premières branches, par exemple quatre premières branches. Dans ce cas, la partie intermédiaire comporte plusieurs portions de raccordement respectives agencées pour raccorder les premières branches au moins deux à deux. Ainsi, une telle première partie d'ancrage permet de réaliser un ancrage tridimensionnel, qui peut avoir une très grande résistance mécanique et une excellente stabilité.

Selon un mode de réalisation de l'invention, la partie intermédiaire est déformable plastiquement de sorte qu'une déformation plastique de la partie intermédiaire rapprochant la première portion de liaison et la deuxième portion de liaison déplace les premières branches et/ou les deuxièmes branches de leur(s) position(s) d'introduction à leur(s) position(s) d'ancrage.

En d'autres termes, une contrainte mécanique appliquée à la partie intermédiaire est déterminée de façon à produire une déformation permanente ou irréversible de la partie intermédiaire.

Ainsi, cette déformation plastique, qui est par définition irréversible et permanente, permet à un chirurgien de placer facilement les premières branches et/ou les deuxièmes branches en position(s) d'ancrage. De plus, le chirurgien dispose de tout le temps nécessaire pour positionner précisément l'implant chirurgical dans la première portion d'os et dans la deuxième portion d'os, ce qui garantit au patient un ancrage résistant et durable de l'implant chirurgical. Avantageusement, la déformation plastique est réalisée dans le domaine de déformation plastique homogène du matériau composant la partie intermédiaire. Ainsi, une telle déformation plastique homogène évite de fragiliser en striction la partie intermédiaire, laquelle conserve ainsi une grande résistance mécanique.

Selon une variante de l'invention, la partie intermédiaire est déformable plastiquement de sorte qu'une déformation plastique de la partie intermédiaire mettant en contact la première portion de liaison et la deuxième portion de liaison déplace les premières branches de la position d'introduction à la position d'ancrage. En d'autres termes, après la déformation plastique, la première portion de liaison touche la deuxième portion de liaison. Ainsi, lors du serrage par un ancillaire de serrage spécifique, le chirurgien peut sentir à quel moment arrêter le serrage de la partie intermédiaire.

Selon un mode de réalisation de l'invention, en alternative à une déformation plastique, la partie intermédiaire est déformable élastiquement de sorte qu'une détente élastique de la partie intermédiaire rapprochant la première portion de liaison et la deuxième portion de liaison déplace les premières branches de leur(s) position(s) d'introduction à leur(s) position(s) d'ancrage.

En d'autres termes, une contrainte mécanique appliquée à la partie intermédiaire est déterminée de façon à produire une déformation réversible de la partie intermédiaire. La partie intermédiaire est précontrainte avant l'introduction de l'implant chirurgical dans le corps du patient, puis cette précontrainte est relâchée totalement ou partiellement pour atteindre la position d'ancrage dans le corps du patient.

Ainsi, la partie intermédiaire est élastiquement déformable entre i) une position de grande déformation élastique, dans laquelle les premières branches et/ou les deuxièmes branches sont en position(s) d'introduction, et ii) une position de déformation élastique faible ou nulle, dans laquelle les premières branches et/ou les deuxièmes branches sont en position(s) d'ancrage.

Selon une variante de l'invention, l'implant chirurgical est configuré pour coopérer avec des moyens de retenue mobiles entre :
- une position de retenue, dans laquelle les moyens de retenue retiennent la partie intermédiaire élastiquement déformée, et
- une position de libération, dans laquelle les moyens de retenue libèrent la partie intermédiaire de sorte que ladite détente élastique peut intervenir.

Par exemple, les moyens de retenue peuvent comprendre un plot, qui, en position de retenue, est inséré dans ledit au moins un évidement. Lorsque l'on retire le plot, la détente élastique de la partie intermédiaire rapproche la première portion de liaison de la deuxième portion de liaison, ce qui déplace les premières branches et/ou les deuxièmes branches vers leur(s) position(s) d'ancrage.

Selon un mode de réalisation de l'invention, les premières branches ont chacune une forme globalement allongée, et dans lequel les premières branches sont substantiellement parallèles dans leur position d'introduction et forment substantiellement un « V » dans leur position d'ancrage.

En d'autres termes, les premières branches forment un « U » resserré en position d'introduction et un « V » en position d'ancrage. La forme en « V » est définie par un secteur angulaire qui est avantageusement compris entre 20 degrés et 60 degrés.

Selon une variante de l'invention, les deuxièmes branches ont chacune une forme globalement allongée, les deuxièmes branches sont substantiellement parallèles dans leur position d'introduction, et les deuxièmes branches forment substantiellement un « V » dans leur position d'ancrage. En d'autres termes, dans cette variante, l'implant chirurgical a globalement une forme de « H » resserré en positions d'introduction et de « X » en positions d'ancrage.

Ainsi, de telles formes permettent une introduction facile et un ancrage ferme de l'implant chirurgical dans la première portion d'os et dans la deuxième portion d'os.

Selon une variante de l'invention, les premières branches s'étendent suivant une première direction longitudinale, les deuxièmes branches s'étendent suivant une deuxième direction longitudinale, les premières branches et les deuxièmes branches formant un angle compris entre 150 degrés et 180 degrés dans un plan contenant la première direction longitudinale et la deuxième direction longitudinale.

Selon un mode de réalisation de l'invention, la partie intermédiaire comporte en outre i) une première portion de raccordement raccordant au moins deux premières branches, et ii) une deuxième portion de raccordement raccordant au moins deux deuxièmes branches, la première portion de liaison et la deuxième portion de liaison étant agencées respectivement de part et d'autre de la première portion de raccordement et de part et d'autre de la deuxième portion de raccordement.

Dans ce mode de réalisation, la première portion de liaison, la deuxième portion de liaison, la première portion de raccordement et la deuxième portion de liaison définissent ledit au moins un évidement. Ainsi, la partie intermédiaire peut présenter une grande résistance mécanique et permettre une grande précision géométrique de la déformation.

Selon une variante de l'invention, la première portion de liaison et la deuxième portion de liaison définissent au moins un évidement présentant un contour fermé défini par la première portion de liaison, par la deuxième portion de liaison, par la première portion de raccordement et par la deuxième portion de raccordement. Ainsi, un tel évidement fermé permet une grande précision géométrique de la déformation.

Alternativement, la partie intermédiaire présente un évidement ayant un contour ouvert vers la deuxième partie d'ancrage.

Selon une variante de l'invention, ledit au moins un évidement contient un élément plus déformable que la partie intermédiaire. À cet effet, la limite de déformation élastique ou plastique du matériau composant cet élément est inférieure à la limite de déformation plastique du matériau composant la partie intermédiaire. Ainsi, l'élément n'empêche ni ne ralentit la déformation plastique rapprochant la première portion de liaison de la deuxième portion de liaison. Cet élément peut par exemple être composé d'un matériau polymère.

Selon un mode de réalisation de l'invention, au moins un évidement a une forme oblongue, dont la longueur s'étend globalement entre la première partie d'ancrage et la deuxième partie d'ancrage.

Ainsi, un tel évidement oblong nécessite une faible course en déformation plastique, donc un serrage limité de la part du chirurgien.

Selon un mode de réalisation de l'invention, au moins un évidement présente une zone étroite et une zone large qui est plus large que la zone étroite, les largeurs étant mesurées entre la première portion de liaison et la deuxième portion de liaison.

En particulier, les largeurs sont mesurées transversalement à une direction longitudinale selon laquelle s'étendent les premières branches.

Par exemple, cet évidement peut présenter approximativement, en vue du dessus, une forme de poire ou de selle de bicyclette. Ainsi, une telle forme dissymétrique permet un déplacement des premières branches plus grand que le déplacement des deuxièmes branches, de façon à adapter l'implant chirurgical à des indications spécifiques.

Selon un mode de réalisation de l'invention, la première portion de liaison présente une première face qui est convexe et qui est orientée vers la deuxième portion de liaison, la première face ayant un profil formé par un arc de cercle dont le rayon est supérieur à 20 mm, de préférence à 40 mm, et la deuxième portion de liaison présente une deuxième face qui est convexe et qui est orientée vers la première portion de liaison, la deuxième face ayant un profil formé par un arc de cercle dont le rayon est supérieur à 20 mm, de préférence à 40 mm.

Ainsi, de tels arcs de cercle permettent, lors d'une déformation plastique, d'obtenir une grande précision géométrique dans le déplacement des premières branches et, le cas échéant, des deuxièmes branches.

Avantageusement, les rayons des profils circulaires sont égaux.

Selon une variante de l'invention, la première distance d'introduction est comprise entre 0 et 2 mm, et dans lequel la première distance d'ancrage est comprise entre 1 et 10 mm.

Ainsi, de telles dimensions sont particulièrement adaptées aux articulations interphalangiennes des pieds ou des mains.

Selon une variante de l'invention, la deuxième distance d'introduction est comprise entre 0 et 2 mm, et dans lequel la deuxième distance d'ancrage est comprise entre 1 et 10 mm.

Selon un mode de réalisation de l'invention, au moins la partie intermédiaire est composée d'un matériau sélectionné dans le groupe constitué :
- d'un alliage de titane, de préférence de grade 1, 2, 3 ou 4, suivant la norme ISO 5832-2 ou ASTM F67, et
- d'un acier inoxydable suivant la norme ISO 5832-1.

Par exemple, la partie intermédiaire en un tel alliage de titane présente une plage de déformation plastique avec une grande limite à la rupture et une limite d'élasticité relativement petite, ce qui permet d'obtenir la déformation plastique avec un effort de compression peu élevé.

Selon une variante de l'invention, au moins une parmi les premières branches et les deuxièmes branches présente, sur une face externe respective, des crans configurés pour l'ancrage de l'implant chirurgical respectivement dans la première portion d'os et dans la deuxième portion d'os.

Avantageusement, sur la ou chaque portion d'extrémité d'une première ou deuxième branche respective, des crans sont en forme de harpon.

Selon un mode de réalisation de l'invention, la partie intermédiaire comporte en outre au moins deux butées s'étendant en saillie sur deux faces externes opposées de la partie intermédiaire.

Ainsi, les butées permettent de positionner précisément un ancillaire de serrage sur la partie intermédiaire.

Selon une variante de l'invention, dans le cas où la deuxième partie d'ancrage comprend des deuxièmes branches, la partie intermédiaire comporte quatre butées, à savoir deux butées situées du côté des premières branches et deux butées situées du côté des deuxièmes branches. Avantageusement, les butées situées du côté des premières branches sont plus grandes que les butées situées du côté des deuxièmes branches.

Avantageusement, des faces externes de la partie intermédiaire présentent, à côté des butées, des moyens de friction configurés pour agripper par frottement des mors d'un ancillaire de serrage. Par exemple, ces moyens de friction peuvent comprendre des crans ou des rugosités.

Par ailleurs, la présente invention a pour objet un ancillaire de serrage, configuré pour serrer au moins un implant chirurgical selon l'invention, l'ancillaire de serrage comprenant au moins deux mors et au moins une articulation reliant lesdits au moins deux mors, l'ancillaire de serrage étant caractérisé en ce que chaque mors présente un logement respectif de forme complémentaire à une portion de liaison respective de façon à agripper une face externe respective de la partie intermédiaire, et en ce que ladite au moins une articulation est configurée de sorte qu'un déplacement des mors induit un rapprochement de la première portion de liaison et de la deuxième portion de liaison.

Ainsi, un tel ancillaire de serrage permet de serrer l'implant chirurgical jusqu'à obtenir la déformation plastique précitée.

Selon un mode de réalisation de l'invention, ladite au moins une articulation comporte un loquet présentant au moins un cran de verrouillage, l'articulation étant mobile entre :
- une position ouverte, dans laquelle les mors définissent un espace permettant l'installation de l'implant chirurgical,
- une position de retenue, dans laquelle les mors agrippent les faces externes respectives de la partie intermédiaire de sorte que l'ancillaire de serrage retient l'implant chirurgical,
- une position de serrage, dans laquelle les mors serrent la partie intermédiaire de façon à obtenir ledit rapprochement.

Selon un mode de réalisation de l'invention, le loquet est lié à l'un des mors par une liaison pivot, et le loquet présente une portion de manipulation, configurée pour permettre à un chirurgien de manipuler le loquet de façon à mouvoir l'articulation entre la position ouverte, la position de retenue et la position de serrage.

Les modes de réalisation et les variantes mentionnés ci-avant peuvent être pris isolément ou selon toute combinaison techniquement admissible.

La présente invention sera bien comprise et ses avantages ressortiront aussi à la lumière de la description qui va suivre, donnée uniquement à titre d'exemple non limitatif et faite en référence aux dessins annexés, dans lesquels :
- la figure 1 est une vue de face d'un implant chirurgical conforme à l'invention, en configuration d'introduction ;
- la figure 2 est une vue en perspective de l'implant chirurgical de la figure 1, en configuration d'introduction ;
- la figure 3 est une vue de face de l'implant chirurgical de la figure 1, en configuration d'ancrage ;
- la figure 4 est une vue à plus grande échelle du détail IV à la figure 1 ;
- la figure 5 est une vue à plus grande échelle du détail V à la figure 3 ;
- la figure 6 est une vue de côté de l'implant chirurgical de la figure 1 ;
- la figure 7 est une vue similaire à la figure 6 d'un implant chirurgical conforme à une variante de l'invention ;
- la figure 8 est une vue en perspective d'un ancillaire de serrage conforme à l'invention et configuré pour maintenir puis serrer l'implant chirurgical de la figure 1 ;
- la figure 9 est une vue en perspective de l'ancillaire de serrage de la figure 8 retenant l'implant chirurgical de la figure 1 ;
- la figure 10 est une vue à plus grande échelle du détail X à la figure 9 ;
- les figures 11A et 11B sont des vues schématiques et à plus grande échelle du centre de la figure 4 ; les figures 11A et 11B illustrent deux positions différentes pour le serrage de l'implant chirurgical ;
- la figure 12 est une vue de côté d'une partie de l'ancillaire de serrage de la figure 8 dans une position ouverte ;
- la figure 13 est une vue similaire à la figure 12 illustrant l'ancillaire de serrage de la figure 12 dans une position de retenue ;
- la figure 14 est une vue similaire à la figure 12 illustrant l'ancillaire de serrage de la figure 12 dans une position suivant la position de serrage de la figure 13 ;
- la figure 15 est une vue similaire à la figure 12 illustrant l'ancillaire de serrage de la figure 12 dans une position de serrage ;
- la figure 16 est une vue schématique de l'implant chirurgical de la figure 1 en configuration d'introduction ; et
- la figure 17 est une vue similaire à la figure 16 illustrant l'implant chirurgical de la figure 1 en configuration d'ancrage.

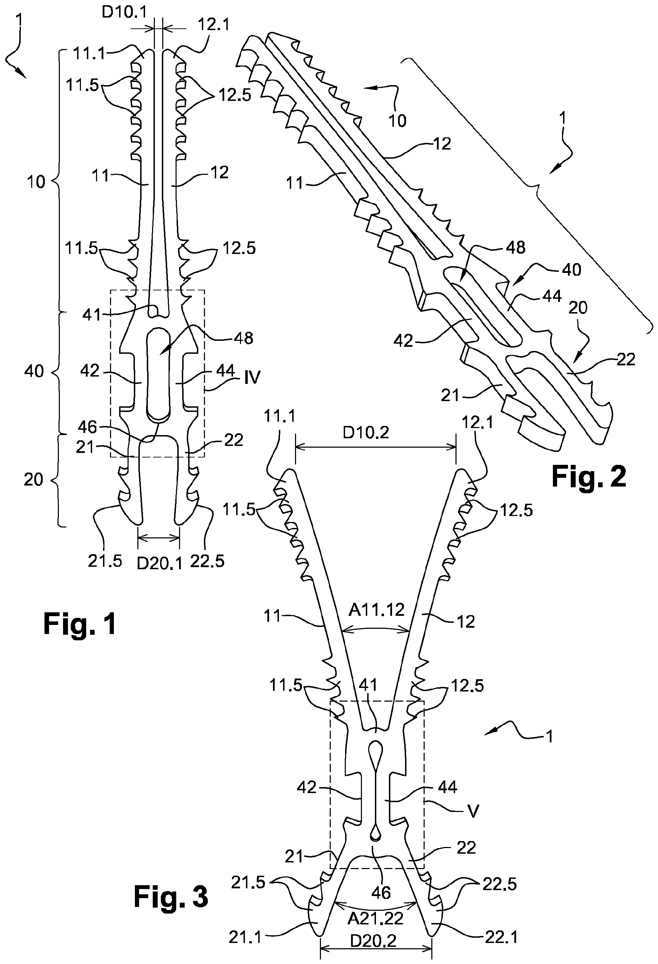

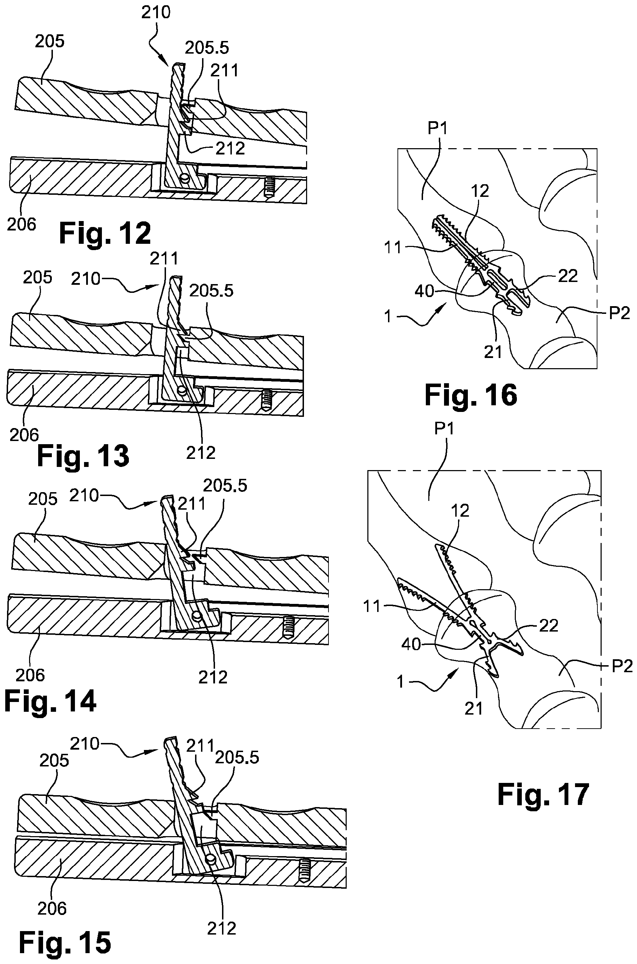

Les figures 1, 2, 3, 4, 5 et 6 illustrent un implant chirurgical 1 destiné à permettre une fusion entre une première portion d'os et une deuxième portion d'os. Dans l'exemple des figures 16 et 17, la première portion d'os et la deuxième portion d'os sont respectivement une phalange proximale P1 et une phalange moyenne P2 d'un orteil.

L'implant chirurgical 1 comprend une première partie d'ancrage 10 et une deuxième partie d'ancrage 20, qui sont respectivement configurées pour être ancrées dans la phalange proximale P1 et dans la phalange moyenne P2.

La première partie d'ancrage 10 comporte deux premières branches 11 et 12. Les premières branches 11 et 12 sont déplaçables entre une position d'introduction (figures 1, 2, 4, 16) et une position d'ancrage (figures 3, 5, 17).

En position d'introduction (figures 1, 4, 16), les extrémités respectives 11.1 et 12.1 des premières branches 11 et 12 définissent une première distance d'introduction D10.1. La première distance d'introduction D10.1 est relativement petite, de façon à permettre l'introduction des premières branches 11 et 12 à l'intérieur de la phalange proximale P1, comme le montre la figure 16. Dans l'exemple des figures 1 à 6, la première distance d'introduction D10.1 est environ égale à 0,4 mm et la première distance d'ancrage D10.2 est environ égale à 6 mm.

En position d'ancrage (figures 3, 5, 17), les extrémités respectives 11.1 et 12.1 des premières branches 11 et 12 définissent une première distance d'ancrage D10.2 qui est plus grande que la première distance d'introduction D10.1. Comme la première distance d'ancrage D10.2 est relativement grande, les premières branches 11 et 12 sont adaptées pour ancrer la première partie d'ancrage 10 à l'intérieur de la phalange proximale P1.

Les premières branches 11 et 12 ont chacune une forme globalement allongée et sont substantiellement parallèles dans leur position d'introduction (figures 1, 4, 16) et forment substantiellement un « U » resserré dans leur position d'introduction (figures 1, 4, 16). Les premières branches 11 et 12 forment substantiellement un « V » dans leur position d'ancrage (figures 3, 5, 17), en l'occurrence un secteur angulaire A11.12 d'environ 30 degrés.

De plus, l'implant chirurgical 1 comprend une partie intermédiaire 40 qui relie la première partie d'ancrage 10 à la deuxième partie d'ancrage 20. La partie intermédiaire 40 s'étend entre la première partie d'ancrage 10 et la deuxième partie d'ancrage 20.

La deuxième partie d'ancrage 20 comporte deux deuxièmes branches 21 et 22. Donc l'implant chirurgical 1 a quatre branches 11, 12, 21 et 22, à raison de deux de chaque côté de la partie intermédiaire 40. Les deuxièmes branches 21 et 22 sont déplaçables entre une position d'introduction (figures 1, 4, 16) et une position d'ancrage (figures 3, 5, 17).

Les deuxièmes branches 21 et 22 ont chacune une forme globalement allongée et sont substantiellement parallèles dans leur position d'introduction (figures 1, 4, 16) et forment substantiellement un « U » resserré dans leur position d'introduction (figures 1, 4, 16). Les deuxièmes branches 21 et 22 forment substantiellement un « V » dans leur position d'ancrage (figures 3, 5, 17), en l'occurrence un secteur angulaire A21.22 d'environ 40 degrés.

Donc l'implant chirurgical 1 a globalement une forme de « H » resserré lorsque les premières branches 11 et 12 et les deuxièmes branches 21 et 22 sont dans leurs positions d'introduction (figures 1, 4, 16), puis de « X » lorsque les premières branches 11 et 12 et les deuxièmes branches 21 et 22 sont dans leurs positions d'ancrage (figures 3, 5, 17).

En position d'introduction (figures 1, 4, 16), les extrémités respectives 21.1 et 22.1 des deuxièmes branches 21 et 22 définissent une deuxième distance d'introduction D20.1. La deuxième distance d'introduction D20.1 est relativement petite, de façon à permettre l'introduction des deuxièmes branches 21 et 22 à l'intérieur de la phalange moyenne P2. Dans l'exemple des figures 1 à 6, la deuxième distance d'introduction D20.1 est environ égale à 1,5 mm et la deuxième distance d'ancrage D20.2 est environ égale à 4,3 mm.

En position d'ancrage (figures 3, 5, 17), les extrémités respectives 21.1 et 22.1 des deuxièmes branches 21 et 22 définissent une deuxième distance d'ancrage D20.2 qui est plus grande que la deuxième distance d'introduction D20.1. Comme la deuxième distance d'ancrage D20.2 est relativement grande, les deuxièmes branches 21 et 22 sont adaptées pour ancrer la deuxième partie d'ancrage 20 à l'intérieur de la phalange moyenne P2.

La partie intermédiaire 40 comporte :
- une première portion de raccordement 41 qui raccorde les deux premières branches 11 et 12,
- une première portion de liaison 42, qui est disposée entre la première partie d'ancrage 10 et la deuxième partie d'ancrage 20, et
- une deuxième portion de liaison 44, qui est disposée entre la première partie d'ancrage 10 et la deuxième partie d'ancrage 20.

La première portion de liaison 42 et la deuxième portion de liaison 44 sont agencées de part et d'autre de l'évidement 48 de sorte que la première portion de liaison 42 est distante de la deuxième portion de liaison 44. La première portion de liaison 42 s'étend substantiellement entre la première branche 11 et la deuxième partie d'ancrage 20. La deuxième portion de liaison 44 s'étend substantiellement entre la deuxième branche 12 et la deuxième partie d'ancrage 20.

La partie intermédiaire 40 comporte en outre un évidement 48 qui présente ici un contour fermé. Dans l'exemple des figures 1 à 6, l'évidement 48 a une forme oblongue, dont la longueur L48 s'étend globalement entre la première partie d'ancrage 10 et la deuxième partie d'ancrage 20.

La partie intermédiaire 40 comporte en outre une deuxième portion de raccordement 46 qui raccorde les deuxièmes branches 21 et 22. La première portion de liaison 42 et la deuxième portion de liaison 44 sont agencées de part et d'autre de l'évidement 48. La première portion de liaison 42 et la deuxième portion de liaison 44 sont agencées respectivement de part et d'autre de la première portion de raccordement 41 et de part et d'autre de la deuxième portion de raccordement 46.

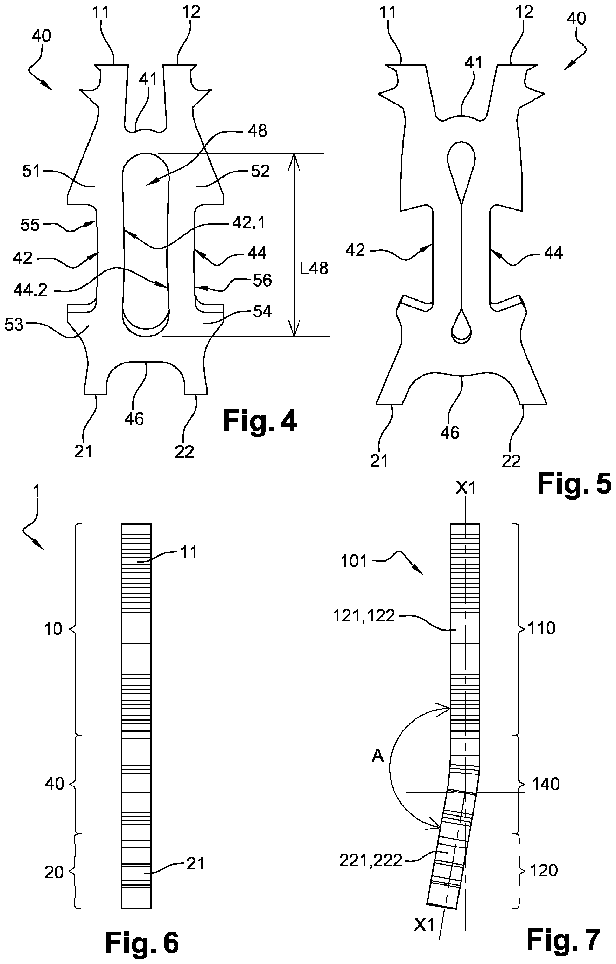

Comme le montrent les figures 1 et 4, le contour fermé de l'évidement 48 est défini par la première portion de liaison 42, par la deuxième portion de liaison 44, par la première portion de raccordement 41 et par la deuxième portion de raccordement 46.

La partie intermédiaire 40 est conformée de sorte qu'un rapprochement de la première portion de liaison 42 et de la deuxième portion de liaison 44 déplace les premières branches 11 et 12 de leur position d'introduction à leur position d'ancrage.

Dans l'exemple des figures 1 à 6, la partie intermédiaire 40 est déformable plastiquement de sorte qu'une déformation plastique de la partie intermédiaire 40 rapprochant la première portion de liaison 42 de la deuxième portion de liaison 44 déplace les premières branches 11 et 12 de leur position d'introduction (figures 1, 4, 16) à leur position d'ancrage (figures 3, 5, 17).

Dans l'exemple des figures 1 à 5, la déformation plastique de la partie intermédiaire 40 met en contact la première portion de liaison 42 et la deuxième portion de liaison 44, ce qui déplace les premières branches 11 et 12 de la position d'introduction (figures 1, 4, 16) à la position d'ancrage (figures 3, 5, 17). Comme le montrent les figures 3 et 5, après la déformation plastique, la première portion de liaison 42 touche la deuxième portion de liaison 44.

La partie intermédiaire 40 est composée d'un alliage de titane de grade 2 (titane T40) suivant la norme ISO 5832-2 ou ASTM F67. La déformation plastique est ici réalisée dans le domaine de déformation plastique homogène du matériau composant la partie intermédiaire 40.

La comparaison des figures 4 et 5 montre le rapprochement mettant en contact la première portion de liaison 42 et la deuxième portion de liaison 44 résultant de la déformation plastique de la partie intermédiaire 40.

De manière similaire au déplacement des premières branches 11 et 12, la partie intermédiaire 40 est déformable plastiquement de sorte qu'une déformation plastique de la partie intermédiaire 40 rapprochant la première portion de liaison 42 de la deuxième portion de liaison 44 déplace les deuxièmes branches 21 et 22 de leur position d'introduction (figures 1, 4, 16) à leur position d'ancrage (figures 3, 5, 17).

Les premières branches 11 et 12 sont plus longues que les deuxièmes branches 21 et 22. Le rapport entre une longueur des premières branches 11 et 12 et une longueur des deuxièmes branches 21 et 22 est compris ici environ égal à 3. Les premières branches 11 et 12 ont la même longueur. Les deuxièmes branches 21 et 22 ont la même longueur.

La longueur de chaque première branche 11 ou 12 est ici environ égale à 11 mm. La longueur de chaque deuxième branche 21 ou 22 est ici environ égale à 4 mm. La partie intermédiaire 40 a ici une longueur, mesurée suivant la direction longitudinale d'une première branche 11 ou 12, environ égale à 5 mm. La partie intermédiaire 40, la première partie d'ancrage 10 et la deuxième partie d'ancrage 20 ont une épaisseur E1 environ égale à 1,5 mm.

Chaque première branche 11 ou 12 et chaque deuxième branche 21 ou 22 présente, sur une face externe respective, des crans 11.5, 12.5 et 21.5, 22.5, qui sont configurés pour l'ancrage de l'implant chirurgical 1 respectivement dans la phalange proximale P1 et dans la phalange moyenne P2. Sur chaque portion d'extrémité d'une première 11, 12 ou deuxième 21, 22 branche respective, des crans 11.5, 12.5, 21.5 et 22.5 sont en forme de harpons.

Dans l'exemple des figures 1 à 6, l'implant chirurgical 1 est monobloc. Donc, comme la partie intermédiaire 40, les premières branches 11 et 12 et les deuxièmes branches 21 et 22 sont composées d'un alliage de titane de grade 2 suivant la norme ISO 5832-2 ou ASTM F67.

Comme le montrent les figures 3 et 5, après la déformation plastique, la première portion de liaison 42 touche la deuxième portion de liaison 44, si bien que l'évidement 48 est très réduit voire inexistant.

La première portion de liaison 42 présente une première face 42.1 qui est convexe et qui est orientée vers la deuxième portion de liaison 44. La première face 42.1 a un profil formé par un arc de cercle dont le rayon est ici environ égal à 35 mm.

De même, la deuxième portion de liaison 44 présente une deuxième face 44.2 qui est convexe et qui est orientée vers la première portion de liaison 42. La deuxième face 44.2 a un profil formé par un arc de cercle dont le rayon est ici environ égal à 35 mm.

De plus, la partie intermédiaire 40 comporte en outre quatre butées 51, 52, 53 et 54 qui s'étendent en saillie sur deux faces externes respectives 55, 56 de la partie intermédiaire 40 qui sont opposées. Les butées 51 et 52 s'étendent en saillie sur la face externe 55, et butées 53 et 54 s'étendent en saillie sur la face externe 56. Les deux butées 51 et 52 sont situées du côté des premières branches 11 et 12, tandis que les butées 53 et 54 sont situées du côté des deuxièmes branches 21 et 22.

Chaque face externe 55 ou 56 de la partie intermédiaire 40 présente, à côté des butées 51, 52, 53, 54, des moyens de friction non représentés, crans ou rugosités, qui sont configurés pour agripper par frottement des mors d'un ancillaire de serrage.

La figure 7 illustre un implant chirurgical 101 conforme à une variante de l'invention. L'implant chirurgical 101 est semblable à l'implant chirurgical 1, à l'exception des différences notables mentionnées ci-après. L'implant chirurgical 101 comprend une première partie d'ancrage 110, une deuxième partie d'ancrage 120 et une partie intermédiaire 140. La première partie d'ancrage 110 comporte deux premières branches 111 et 112 semblables aux premières branches 11 et 12 de l'implant chirurgical 1. La deuxième partie d'ancrage 120 comporte deux deuxièmes branches 121 et 122 semblables aux deuxièmes branches 11 et 12 de l'implant chirurgical 1.

L'implant chirurgical 101 diffère de l'implant chirurgical 1, car les premières branches 111 et 112 s'étendent suivant une première direction longitudinale X1, tandis que les deuxièmes branches 121 et 122 s'étendent suivant une deuxième direction longitudinale X2. Ainsi, les premières branches 111 et 112 et les deuxièmes branches 121 et 122 forment un angle A entre elles.

L'angle A est ici environ égal à 170 degrés dans un plan contenant la première direction longitudinale X1 et la deuxième direction longitudinale X2. L'angle A est ici représenté dans le plan de la figure 7, qui est parallèle au plan contenant la première direction longitudinale X1 et la deuxième direction longitudinale X2.

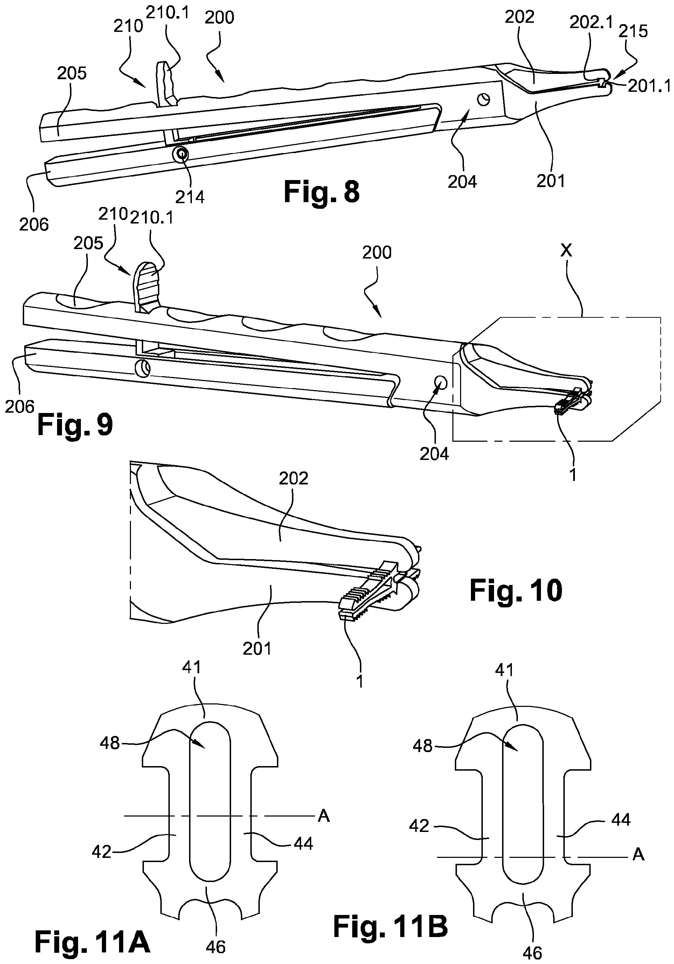

Les figures 8, 9, 10, 12, 13, 14 et 15 illustrent un ancillaire de serrage 200 conforme à l'invention, qui est configuré pour serrer l'implant chirurgical 1 ou 101. L'ancillaire de serrage 200 comprend deux mors 201 et 202 et une articulation 204. L'articulation 204 relie les mors 201 et 202. L'ancillaire de serrage 200 comprend en outre deux barres de préhension 205 et 206. Chaque barre de préhension 205 ou 206 est solidaire d'un mors respectif 201 ou 202.

Chaque mors 201 ou 202 définit un logement respectif 201.1 ou 202.1 de forme complémentaire à une portion de liaison respective 42 ou 44, de sorte que chaque mors 201 ou 202 peut agripper une face externe respective 55 ou 56 de la partie intermédiaire 40. Dans l'exemple des figures 1 à 6, la première portion de liaison 42 et la deuxième portion de liaison 44 ont chacune une section à profil rectangulaire. Donc, dans l'exemple des figures 8 à 15, chaque logement respectif 201.1 ou 202.1 défini par les mors 201 et 202 est globalement parallélépipédique à profil rectangulaire ouvert sur un côté, comme le montre la figure 8.

L'articulation 204 est configurée de sorte qu'un déplacement des mors 202 et 204 induit une déformation plastique de la partie intermédiaire 40 rapprochant la première portion de liaison 42 de la deuxième portion de liaison 44.

L'articulation 204 comporte un loquet 210 qui présente deux crans de verrouillage 211 et 212. Le mors 205 présente un ergot 205.5 configuré pour coopérer avec les crans de verrouillage 211 et 212 par complémentarité de formes. Le loquet 210 est lié au mors 206 par une liaison pivot 214. Le loquet 210 présente une portion de manipulation 210.1, qui est configurée pour permettre à un chirurgien de manipuler le loquet 210 de façon à mouvoir l'articulation 204.

L'articulation 204 est mobile entre :
- une position ouverte (figures 8 et 12), dans laquelle les mors 201 et 202 définissent un espace 215 permettant l'installation de l'implant chirurgical 1 ; en position ouverte, l'ergot 205.5 ne coopère pas encore avec les crans de verrouillage 211 et 212 ;
- une position de retenue (figures 9, 10 et 13), dans laquelle les mors 201 et 202 agrippent les faces externes respectives 55 et 56 de la partie intermédiaire 40 de sorte que l'ancillaire de serrage 200 retient l'implant chirurgical 1 ; en position de retenue, l'ergot 205.5 est engagé entre les crans de verrouillage 211 et 212 ;
- une position de serrage (figure 15), dans laquelle les mors 201 et 202 serrent la partie intermédiaire 40 de façon à atteindre la déformation plastique prévue ; en position de serrage, l'ergot 205.5 est dégagé des crans de verrouillage 211 et 212, de sorte que l'ergot 205.5 se trouve sous les crans de verrouillage 211 et 212.

Grâce à la portion de manipulation 210.1, le chirurgien peut manipuler le loquet 210 de façon à mouvoir l'articulation 204 entre la position ouverte (figures 8 et 12), la position de retenue (figures 9, 10 et 13) et la position de serrage (figure 15). La figure illustre l'ancillaire de serrage 200 dans une position suivant la position de serrage (figure 13).

La figure 11A illustre une première position de serrage de l'implant chirurgical 1 par rapport à l'ancillaire 200. La résultante des efforts de serrage exercés par les mors 201 et 202 sur l'implant chirurgical 1 est portée globalement par un axe de résultante A.

Dans cette première position de serrage, l'axe de résultante A passe sensiblement par le milieu de l'évidement 48, suivant une direction longitudinale globalement définie par les premières branches 11 et 12 en positions d'introduction (figures 1, 2 et 4).

Dans cette première position de serrage, les efforts de serrage exercés par les mors 201 et 202 sur l'implant chirurgical 1 sont répartis uniformément sur la partie intermédiaire 40. Ainsi, la déformation plastique des premières branches et des deuxièmes branches pourrait être symétrique dans le cas où les premières branches et les deuxièmes branches sont symétriques.

La figure 11B illustre une deuxième position de serrage de l'implant chirurgical 1 par rapport à l'ancillaire 200. La résultante des efforts de serrage exercés par les mors 201 et 202 sur l'implant chirurgical 1 est portée globalement par l'axe de résultante A.

Dans cette deuxième position de serrage, à la différence de la première position de serrage illustrée par la figure 11A, l'axe de résultante A ne passe pas par le milieu de l'évidement 48, mais plus près de la deuxième partie d'ancrage 20.

Dans cette deuxième position de serrage, les efforts de serrage exercés par les mors 201 et 202 sur l'implant chirurgical 1 ne sont pas répartis uniformément sur la partie intermédiaire 40. Ainsi, la déformation plastique des premières branches et des deuxièmes branches n'est pas symétrique. Les deuxièmes branches seraient plus écartées que les premières branches.

Les figures 16 et 17 illustrent l'implant chirurgical 1 respectivement avant et après déformation plastique de la partie intermédiaire 40, donc respectivement avant et après écartement des premières branches 11 et 12 et des deuxièmes branches 21 et 22. La figure 16 illustre donc les premières branches 11 et 12 et les deuxièmes branches 21 et 22 dans leurs positions d'introduction, tandis que la figure 17 illustre les premières branches 11 et 12 et les deuxièmes branches 21 et 22 dans leurs positions d'ancrage.

Bien entendu, la présente invention n'est pas limitée aux exemples particuliers décrits dans la présente demande. D'autres modes de réalisation à la portée de l'homme du métier peuvent aussi être envisagés sans sortir du cadre de la présente invention.

## Revendications

1. Implant chirurgical (1), destiné à permettre une fusion entre une première portion d'os (P1) et une deuxième portion d'os (P2), l'implant chirurgical (1) comprenant au moins :
- une première partie d'ancrage (10) configurée pour être ancrée dans la première portion d'os (P1), la première partie d'ancrage (10) comportant au moins deux premières branches (11, 12), les premières branches (11, 12) étant déplaçables entre :
• une position d'introduction, dans laquelle les extrémités respectives des premières branches (11, 12) définissent une première distance d'introduction (D10.1), de façon à permettre l'introduction des premières branches (11, 12) à l'intérieur de la première portion d'os (P1), et
• une position d'ancrage, dans laquelle les extrémités respectives des premières branches (11, 12) définissent une première distance d'ancrage (D10.2) plus grande que la première distance d'introduction (D10.1), de sorte que les premières branches (11, 12) sont adaptées pour ancrer la première partie d'ancrage (10) à l'intérieur de la première portion d'os (P1),
- une deuxième partie d'ancrage (20) configurée pour être ancrée dans la deuxième portion d'os (P2),
- une partie intermédiaire (40) reliant la première partie d'ancrage (10) à la deuxième partie d'ancrage (20) ;
l'implant chirurgical (1) étant **caractérisé en ce que** :
- la partie intermédiaire (40) comporte au moins :
• un évidement (48),
• une première portion de liaison (42) disposée entre la première partie d'ancrage (10) et la deuxième partie d'ancrage (20), et
• une deuxième portion de liaison (44) disposée entre la première partie d'ancrage (10) et la deuxième partie d'ancrage (20), la première portion de liaison (42) et la deuxième portion de liaison (44) étant agencées de part et d'autre dudit au moins un évidement (48) de sorte que la première portion de liaison (42) est distante de la deuxième portion de liaison (44), et
- la partie intermédiaire (40) est conformée de sorte qu'un rapprochement de la première portion de liaison (42) et de la deuxième portion de liaison (44) déplace les premières branches (11, 12) de leur position d'introduction à leur position d'ancrage.

2. Implant chirurgical (1) selon la revendication 1, dans lequel la deuxième partie d'ancrage (20) comporte au moins deux deuxièmes branches (21, 22), les deuxièmes branches (21, 22) étant déplaçables entre :
• une position d'introduction, dans laquelle les extrémités respectives des deuxièmes branches (21, 22) définissent une deuxième distance d'introduction (D20.1), de façon à permettre l'introduction des deuxièmes branches (21, 22) à l'intérieur de la deuxième portion d'os (P2), et
• une position d'ancrage, dans laquelle les extrémités respectives des deuxièmes branches (21, 22) définissent une deuxième distance d'ancrage (D20.2) plus grande que la deuxième distance d'introduction (D20.1), de sorte que les deuxièmes branches (21, 22) sont adaptées pour ancrer la deuxième partie d'ancrage (20) à l'intérieur de la deuxième portion d'os (P2),
dans lequel la partie intermédiaire (40) est conformée de sorte qu'un rapprochement de la première portion de liaison (42) et de la deuxième portion de liaison (44) déplace les deuxièmes branches (21, 22) de leur position d'introduction à leur position d'ancrage.

3. Implant chirurgical (1) selon la revendication 1 ou 2, dans lequel la partie intermédiaire (40) est déformable plastiquement de sorte qu'une déformation plastique de la partie intermédiaire (40) rapprochant la première portion de liaison (42) et la deuxième portion de liaison (44) déplace les premières branches (11, 12) et/ou les deuxièmes branches (21, 22) de leur(s) position(s) d'introduction à leur(s) position(s) d'ancrage.

4. Implant chirurgical selon la revendication 1 ou 2, dans lequel la partie intermédiaire est déformable élastiquement de sorte qu'une détente élastique de la partie intermédiaire rapprochant la première portion de liaison et la deuxième portion de liaison déplace les premières branches et/ou les deuxièmes branches de leur(s) position(s) d'introduction à leur(s) position(s) d'ancrage.

5. Implant chirurgical (1) selon l'une quelconque des revendications précédentes, dans lequel les premières branches (11, 12) ont chacune une forme globalement allongée, et dans lequel les premières branches (11, 12) sont substantiellement parallèles dans leur position d'introduction et forment substantiellement un « V » dans leur position d'ancrage.

6. Implant chirurgical (1) selon la revendication 2, dans lequel la partie intermédiaire (40) comporte en outre une première portion de raccordement (41) raccordant au moins deux premières branches (11, 12), une deuxième portion de raccordement (46) raccordant au moins deux deuxièmes branches (21, 22), la première portion de liaison (42) et la deuxième portion de liaison (44) étant agencées respectivement de part et d'autre de la première portion de raccordement (41) et de part et d'autre de la deuxième portion de raccordement (46).

7. Implant chirurgical (1) selon l'une quelconque des revendications précédentes, dans lequel au moins un évidement (48) a une forme oblongue, dont la longueur s'étend globalement entre la première partie d'ancrage (10) et la deuxième partie d'ancrage (20).

8. Implant chirurgical selon l'une quelconque des revendications précédentes, dans lequel au moins un évidement présente une zone étroite et une zone large qui est plus large que la zone étroite, les largeurs étant mesurées entre la première portion de liaison et la deuxième portion de liaison.

9. Implant chirurgical (1) selon l'une quelconque des revendications précédentes, dans lequel la première portion de liaison (42) présente une première face (42.1) qui est convexe et qui est orientée vers la deuxième portion de liaison (44), la première face (42.1) ayant un profil formé par un arc de cercle dont le rayon est supérieur à 20 mm, de préférence à 40 mm, et dans lequel la deuxième portion de liaison (44) présente une deuxième face (44.2) qui est convexe et qui est orientée vers la première portion de liaison (42), la deuxième face (44.2) ayant un profil formé par un arc de cercle dont le rayon est supérieur à 20 mm, de préférence à 40 mm.

10. Implant chirurgical (1) selon l'une quelconque des revendications précédentes, dans lequel au moins la partie intermédiaire (40) est composée d'un matériau sélectionné dans le groupe constitué :
• d'un alliage de titane, de préférence de grade 1, 2, 3 ou 4, suivant la norme ISO 5832-2 ou ASTM F67, et
• d'un acier inoxydable suivant la norme ISO 5832-1.

11. Implant chirurgical (1) selon l'une quelconque des revendications précédentes, dans lequel la partie intermédiaire (40) comporte en outre au moins deux butées (51, 52, 53, 54) s'étendant en saillie sur deux faces externes (55, 56) opposées de la partie intermédiaire (40).

12. Ancillaire de serrage (200, 300), configuré pour serrer au moins un implant chirurgical (1) selon l'une quelconque des revendications précédentes, l'ancillaire de serrage (200, 300) comprenant au moins deux mors (201, 202 ; 301, 302) et au moins une articulation (204) reliant lesdits au moins deux mors (201, 202 ; 301, 302), l'ancillaire de serrage (200) étant **caractérisé en ce que** chaque mors (201, 202; 301, 302) présente un logement respectif (201.1, 202.1 ; 301.1, 301.2, 302.1, 302.2) de forme complémentaire à une portion de liaison respective (40) de façon à agripper une face externe respective (55, 56) de la partie intermédiaire (40), et **en ce que** ladite au moins une articulation (204) est configurée de sorte qu'un déplacement des mors (201, 202 ; 301, 302) induit un rapprochement de la première portion de liaison (42) et de la deuxième portion de liaison (44).

13. Ancillaire de serrage selon la revendication 12, dans lequel ladite au moins une articulation (204) comporte un loquet (210) présentant au moins un cran de verrouillage, l'articulation (204) étant mobile entre :
- une position ouverte, dans laquelle les mors (201, 202 ; 301, 302) définissent un espace (215) permettant l'installation de l'implant chirurgical (1),
- une position de retenue, dans laquelle les mors (201, 202 ; 301, 302) agrippent les faces externes respectives (55, 56) de la partie intermédiaire (40) de sorte que l'ancillaire de serrage (200, 300) retient l'implant chirurgical (1),
- une position de serrage, dans laquelle les mors (201, 202 ; 301, 302) serrent la partie intermédiaire (40) de façon à obtenir ledit rapprochement.

14. Ancillaire de serrage (200, 300) selon la revendication 13, dans lequel le loquet est lié à l'un des mors (201, 202 ; 301, 302) par une liaison pivot (214), et dans lequel le loquet (210) présente une portion de manipulation (210.1), configurée pour permettre à un chirurgien de manipuler le loquet (210) de façon à mouvoir l'articulation (204) entre la position ouverte, la position de retenue et la position de serrage.

## Patentansprüche

1. Chirurgisches Implantat (1), das ausgelegt ist, um eine Fusion zwischen einem ersten Knochenabschnitt (P1) und einem zweiten Knochenabschnitt (P2) zu ermöglichen, wobei das chirurgische Implantat (1) mindestens Folgendes umfasst:
- einen ersten Verankerungsteil (10), der konfiguriert ist, um im ersten Knochenabschnitt (P1) verankert zu sein, wobei der erste Ankerteil (10) mindestens zwei erste Schenkel (11, 12) umfasst, wobei die ersten Schenkel (11, 12) verschiebbar sind zwischen:
-- einer Einführungsposition, in der die entsprechenden Enden der ersten Schenkel (11, 12) einen ersten Einführungsabstand (D10.1) definieren, um die Einführung der ersten Schenkel (11, 12) in das Innere des ersten Knochenabschnitts (P1) zu ermöglichen, und
-- einer Verankerungsposition, in der die entsprechenden Enden der ersten Schenkel (11, 12) einen ersten Verankerungsabstand (D10.2) definieren, der größer als der erste Einführungsabstand (D10.1) ist, so dass die ersten Schenkel (11, 12) angepasst sind, um den ersten Verankerungsteil (10) im Inneren des ersten Knochenabschnitts (P1) zu verankern,
- einen zweiten Verankerungsteil (20), der konfiguriert ist, um im zweiten Knochenabschnitt (P2) verankert zu sein,
- einen Zwischenteil (40), der den ersten Verankerungsteil (10) mit dem zweiten Verankerungsteil (20) verbindet;
wobei das chirurgische Implantat (1) **dadurch gekennzeichnet ist, dass**:
- der Zwischenteil (40) mindestens Folgendes umfasst:
-- eine Aussparung (48),
-- einen ersten Verbindungsabschnitt (42), der zwischen dem ersten Verankerungsteil (10) und dem zweiten Verankerungsteil (20) angeordnet ist, und
-- einen zweiten Verbindungsabschnitt (44), der zwischen dem ersten Verankerungsteil (10) mit dem zweiten Verankerungsteil (20) angeordnet ist, wobei der erste Verbindungsabschnitt (42) und der zweite Verbindungsabschnitt (44) auf beiden Seiten der mindestens einen Aussparung (48) angebracht sind, so dass der erste Verbindungsabschnitt (42) entfernt vom zweiten Verbindungsabschnitt (44) ist, und
- der Zwischenteil (40) derart ausgebildet ist, dass eine Annäherung des ersten Verbindungsabschnitts (42) und des zweiten Verbindungsabschnitts (44) die ersten Schenkel (11, 12) aus ihrer Einführungsposition in ihre Verankerungsposition verschiebt.

2. Chirurgisches Implantat (1) nach Anspruch 1, wobei der zweite Verankerungsteil (20) mindestens zwei zweite Schenkel (21, 22) umfasst, wobei die zweiten Schenkel (21, 22) verschiebbar sind zwischen:
- einer Einführungsposition, in der die entsprechenden Enden der zweiten Schenkel (21, 22) einen zweiten Einführungsabstand (D20.1) definieren, um die Einführung der zweiten Schenkel (21, 22) in das Innere des zweiten Knochenabschnitts (P2) zu ermöglichen, und
- einer Verankerungsposition, in der die entsprechenden Enden der zweiten Schenkel (21, 22) einen zweiten Verankerungsabstand (D20.2) definieren, der größer als der zweite Einführungsabstand (D20.1) ist, so dass die zweiten Schenkel (21, 22) angepasst sind, um den zweiten Verankerungsteil (20) im Inneren des zweiten Knochenabschnitts (P2) zu verankern,
wobei der Zwischenteil (40) derart ausgebildet ist, dass eine Annäherung des ersten Verbindungsabschnitts (42) und des zweiten Verbindungsabschnitts (44) die zweiten Schenkel (21, 22) aus ihrer Einführungsposition in ihre Verankerungsposition verschiebt.

3. Chirurgisches Implantat (1) nach Anspruch 1 oder 2, wobei der Zwischenteil (40) plastisch derart verformbar ist, dass eine plastische Verformung des Zwischenteils (40) die den ersten Verbindungsabschnitt (42) und den zweiten Verbindungsabschnitt (44) annähert, die ersten Schenkel (11, 12) und/oder die zweiten Schenkel (21, 22) aus ihrer/ihren Einführungsposition(en) in ihre Verankerungsposition(en) verschiebt.

4. Chirurgisches Implantat nach Anspruch 1 oder 2, wobei der Zwischenteil plastisch derart verformbar ist, dass ein Auffedern des Zwischenteils die den ersten Verbindungsabschnitt und des zweiten Verbindungsabschnitts annähert, die ersten Schenkel und/oder die zweiten Schenkel aus ihrer/ihren Einführungsposition(en) in ihre Verankerungsposition(en) verschiebt.

5. Chirurgisches Implantat (1) nach einem der vorhergehenden Ansprüche, wobei die ersten Schenkel (11, 12) jeweils eine im Wesentlichen verlängerte Form aufweisen, und wobei die ersten Schenkel (11, 12) im Wesentlichen parallel in ihrer Einführungsposition sind und im Wesentlichen in ihrer Verankerungsposition ein "V" bilden.

6. Chirurgisches Implantat (1) nach Anspruch 2, wobei der Zwischenteil (40) außerdem einen ersten Anschlussabschnitt (41) umfasst, der mindestens zwei erste Schenkel (11, 12) anschließt, wobei ein zweiter Anschlussabschnitt (46) mindestens zwei zweite Schenkel (21, 22) anschließt, wobei der erste Verbindungsabschnitt (42) und der zweite Verbindungsabschnitt (44) jeweils auf beiden Seiten des ersten Anschlussabschnitts (41) und auf beiden Seiten des zweiten Anschlussabschnitts (46) angebracht sind.

7. Chirurgisches Implantat (1) nach einem der vorhergehenden Ansprüche, wobei mindestens eine Aussparung (48) eine verlängerte Form aufweist, deren Länge sich im Allgemeinen zwischen dem ersten Verankerungsteil (10) und dem zweiten Verankerungsteil (20) erstreckt.

8. Chirurgisches Implantat nach einem der vorhergehenden Ansprüche, wobei mindestens eine Aussparung einen engen Bereich und einen breiten Bereich aufweist, der breiter als der enge Bereich ist, wobei die Breiten zwischen dem ersten Verbindungsabschnitt und dem zweiten Verbindungsabschnitt gemessen werden.

9. Chirurgisches Implantat (1) nach einem der vorhergehenden Ansprüche, wobei der erste Verbindungsabschnitt (42) eine erste Seite (42.1) aufweist, die konvex ist, und die hin zum zweiten Verbindungsabschnitt (44) gerichtet ist, wobei die erste Seite (42.1) ein Profil aufweist, das durch einen Kreisbogen gebildet ist, dessen Radius größer als 20 mm, vorzugsweise als 40 mm ist, und wobei der zweite Verbindungsabschnitt (44) eine zweite Seite (44.2) aufweist, die konvex ist, und die hin zum ersten Verbindungsabschnitt (42) gerichtet ist, wobei die zweite Seite (44.2) ein Profil aufweist, das durch einen Kreisbogen gebildet ist, dessen Radius größer als 20 mm, vorzugsweise als 40 mm ist.

10. Chirurgisches Implantat (1) nach einem der vorhergehenden Ansprüche, wobei mindestens der Zwischenteil (40) aus einem Material zusammengesetzt ist, ausgewählt aus der Gruppe, bestehend aus:
- einer Titanlegierung, vorzugsweise mit dem Grad 1, 2, 3 oder 4, gemäß der Norm ISO 5832-2 oder ASTM F67, und
- einem Edelstahl gemäß der Norm ISO 5832-1.

11. Chirurgisches Implantat (1) nach einem der vorhergehenden Ansprüche, wobei der Zwischenteil (40) außerdem mindestens zwei Anschläge (51, 52, 53, 54) umfasst, die sich vorspringend auf zwei äußeren Seiten (55, 56) erstrecken, die dem Zwischenteil (40) gegenüber liegen.

12. Klemmhilfe (200, 300), die konfiguriert ist, um mindestens ein chirurgisches Implantat (1) nach einem der vorhergehenden Ansprüche zu klemmen, wobei die Klemmhilfe (200, 300) mindestens zwei Backen (201, 202; 301, 302) und mindestens ein Gelenk (204) umfasst, das die mindestens zwei Backen (201, 202 ; 301, 302) verbindet, wobei die Klemmhilfe (200) **dadurch gekennzeichnet ist, dass** jede Backe (201, 202; 301, 302) eine entsprechende Aufnahme (201.1, 202.1; 301.1, 301.2, 302.1, 302.2) mit einer komplementären Form zu einem entsprechenden Verbindungsabschnitt (40) aufweist, um eine entsprechende äußere Seite (55, 56) des Zwischenteils (40) zu greifen, und dadurch, dass das mindestens eine Gelenk (204) derart konfiguriert ist, dass eine Verschiebung der Backen (201, 202; 301, 302) eine Annäherung des ersten Verbindungsabschnitts (42) und des zweiten Verbindungsabschnitts (44) veranlasst.

13. Hilfsklemme nach Anspruch 12, wobei das mindestens eine Gelenk (204) einen Schnapper (210) aufweist, der mindestens eine Verriegelungsrast darstellt, wobei das Gelenk (204) beweglich ist zwischen:
- einer offenen Position, in der die Backen (201, 202; 301, 302) einen Raum (215) definieren, der die Installation des chirurgischen Implantats (1) ermöglicht,
- eine Rückhalteposition, in der die Backen (201, 202; 301, 302) die entsprechenden äußeren Seiten (55, 56) des Zwischenteils (40) derart greifen, dass die Hilfsklemme (200, 300) das chirurgische Implantat (1) zurückhält,
- eine Klemmposition, in der die Backen (201, 202; 301, 302) den Zwischenteil (40) klemmen, um die Annäherung zu erzielen.

14. Hilfsklemme (200, 300) nach Anspruch 13, wobei der Schnapper mit einer der Backen (201, 202; 301, 302) durch eine Schwenkverbindung (214) verbunden ist, und wobei der Schnapper (210) einen Handhabungsabschnitt (210.1) aufweist, der konfiguriert ist, um einem Chirurgen zu ermöglichen, den Schnapper (210) zu handhaben, um das Gelenk (204) zwischen der geöffneten Position, der Rückhalteposition und der Klemmposition zu bewegen.

## Claims

1. A surgical implant (1) intended to allow a fusion between a first bone portion (P1) and a second bone portion (P2), the surgical implant (1) comprising at least:
- a first anchoring part (10) configured to be anchored in the first bone portion (P1), the first anchoring part (10) including at least two first branches (11, 12), the first branches (11, 12) being displaceable between:
• an introduction position, in which the respective ends of the first branches (11, 12) define a first introduction distance (D10.1), so as to allow the introduction of the first branches (11, 12) inside the first bone portion (P1), and
• an anchoring position, in which the respective ends of the first branches (11, 12) define a first anchoring distance (D10.2) larger than the first introduction distance (D10.1), such that the first branches (11, 12) are adapted to anchor the first anchoring part (10) inside the first bone portion (P1),
- a second anchoring part (20) configured to be anchored in the second bone portion (P2),
- an intermediate part (40) linking the first anchoring part (10) to the second anchoring part (20);
the surgical implant (1) being **characterized in that**:
- the intermediate part (40) includes at least:
• a recess (48),
• a first bonding portion (42) disposed between the first anchoring part (10) and the second anchoring part (20), and
• a second bonding portion (44) disposed between the first anchoring part (10) and the second anchoring part (20), the first bonding portion (42) and the second bonding portion (44) being arranged on either side of said at least one recess (48) such that the first bonding portion (42) is spaced apart from the second bonding portion (44), and
- the intermediate part (40) is shaped such that bringing the first bonding portion (42) and the second bonding portion (44) closer to each other, displaces the first branches (11, 12) from their introduction position to their anchoring position.

2. The surgical implant (1) according to claim 1, wherein the second anchoring part (20) includes at least two second branches (21, 22), the second branches (21, 22) being displaceable between:
• an introduction position, in which the respective ends of the second branches (21, 22) define a second introduction distance (D20.1), so as to allow the introduction of the second branches (21, 22) inside the second bone portion (P2), and
• an anchoring position, in which the respective ends of the second branches (21, 22) define a second anchoring distance (D20.2) larger than the second introduction distance (D20.1), such that the second branches (21, 22) are adapted to anchor the second anchoring part (20) inside the second bone portion (P2),
in which the intermediate part (40) is shaped such that bringing the first bonding portion (42) and the second bonding portion (44) closer to each other, displaces the second branches (21, 22) from their introduction position to their anchoring position.

3. The surgical implant (1) according to claim 1 or 2, wherein the intermediate part (40) is plastically deformable such that a plastic deformation of the intermediate part (40) bringing the first bonding portion (42) and the second bonding portion (44) closer to each other, displaces the first branches (11, 12) and/or the second branches (21, 22) from their introduction position(s) to their anchoring position(s).

4. The surgical implant according to claim 1 or 2, wherein the intermediate part is elastically deformable such that an elastic expansion of the intermediate part, bringing the first bonding portion and the second bonding portion closer to each other, displaces the first branches and/or the second branches from their introduction position(s) to their anchoring position(s).

5. The surgical implant (1) according to any one of the preceding claims, wherein the first branches (11, 12) each have a generally elongate shape, and wherein the first branches (11, 12) are substantially parallel in their introduction position and substantially form a « V » in their anchoring position.

6. The surgical implant (1) according to claim 2, wherein the intermediate part (40) further includes a first connecting portion (41) connecting at least two first branches (11, 12), a second connecting portion (46) connecting at least two second branches (21, 22), the first bonding portion (42) and the second bonding portion (44) being respectively arranged on either side of the first connecting portion (41) and on either side of the second connecting portion (46).

7. The surgical implant (1) according to any one of the preceding claims, wherein at least one recess (48) has an oblong shape, whose length extends generally between the first anchoring part (10) and the second anchoring part (20).

8. The surgical implant (1) according to any one of the preceding claims, wherein at least one recess has a narrow zone and a wide zone which is wider than the narrow zone, the widths being measured between the first bonding portion and the second bonding portion.

9. The surgical implant (1) according to any one of the preceding claims, wherein the first bonding portion (42) has a first face (42.1) which is convex and which is oriented toward the second bonding portion (44), the first face (42.1) having a profile formed by an arc of a circle whose radius is greater than 20 mm, preferably 40 mm, and wherein the second bonding portion (44) has a second face (44.2) which is convex and which is oriented toward the first bonding portion (42), the second face (44.2) having a profile formed by an arc of a circle whose radius is greater than 20 mm, preferably than 40 mm.

10. The surgical implant (1) according to any one of the preceding claims, wherein at least the intermediate part (40) is composed of a material selected from the group consisting of:
• a titanium alloy, preferably of grade 1, 2, 3 or 4, according to the standard ISO 5832-2 or ASTM F67, and
• a stainless steel according to the standard ISO 5832-1.

11. The surgical implant (1) according to any one of the preceding claims, wherein the intermediate part (40) further includes at least two stops (51, 52, 53, 54) protruding on two outer faces (55, 56) opposite to the intermediate part (40).

12. A clamping ancillary (200, 300), configured to clamp at least one surgical implant (1) according to any one of the preceding claims, the clamping ancillary (200, 300) comprising at least two jaws (201, 202; 301, 302) and at least one hinge (204) linking said at least two jaws (201, 202; 301, 302), the clamping ancillary (200) being **characterized in that** each jaw (201, 202; 301, 302) has a respective housing (201.1, 202.1, 301.1, 301.2, 302.1, 302.2) of a shape complementary to a respective bonding portion (40) so as to grip a respective outer face (55, 56) of the intermediate part (40), and **in that** said at least one hinge (204) is configured such that a displacement of the jaws (201, 202; 301, 302) induces bringing the first bonding portion (42) and the second bonding portion (44) closer to each other.

13. The clamping ancillary according to claim 12, wherein said at least one hinge (204) includes a latch (210) having at least one locking notch, the hinge (204) being movable between:
- an open position, in which the jaws (201, 202, 301, 302) define a space (215) enabling the installation of the surgical implant (1),
- a retaining position, in which the jaws (201, 202; 301, 302) grip the respective outer faces (55, 56) of the intermediate part (40) such that the clamping ancillary (200, 300) retains the surgical implant (1),
- a clamping position, in which the jaws (201, 202; 301, 302) clamp the intermediate part (40) so as to obtain said approximation.

14. The clamping ancillary (200, 300) according to claim 13, wherein the latch is linked to one of the jaws (201, 202; 301, 302) by a pivot link (214), and wherein the latch (210) has a manipulation portion (210.1) configured to allow a surgeon to manipulate the latch (210) so as to move the hinge (204) between the open position, the retaining position and the clamping position.
